# EUROPEAN PATENT APPLICATION

(11) **EP 4 834 351 A1**
(43) Date of publication of application: **04.11.2026**
(21) Application number: 24913675.5
(22) Date of filing: 27.12.2024
(51) Int. Cl.: A61K 31/7048, A61P 17/14

(54) **PHARMACEUTICAL COMPOSITION COMPRISING ENAVOGLIFLOZIN AS ACTIVE INGREDIENT FOR PREVENTING OR TREATING ALOPECIA AREATA**

(30) Priority: 27.12.2023 KR 20230192684
(71) Applicant: Daewoong Pharmaceutical Co., Ltd., Hwaseong-si, Gyenoggi-do 18623 (KR)
(72) Inventor: PARK, Sun Hwa, Hwaseong-si Gyeonggi-do 18479 (KR); JI, Hye Young, Yongin-si Gyeonggi-do 17010 (KR); CHOI, Ji Soo, Seoul 06310 (KR); PARK, Joon Seok, Yongin-si Gyeonggi-do 17000 (KR); KIM, Beom Joon, Seoul 06044 (KR); SEOK, Joon, Seoul 05502 (KR); LEE, Jung Ok, Seoul 02873 (KR); KIM, Yu Jin, Seoul 07032 (KR); KIM, Su-Young, Seoul 02512 (KR)
(74) Representative: Plasseraud IP
(86) International application number: PCT/KR2024/021275
(87) International publication number: WO 2025/143870

(57) **Abstract**

The present disclosure relates to a pharmaceutical composition for use in preventing or treating alopecia areata, comprising enavogliflozin as an active ingredient. Enavogliflozin of the present disclosure increases BHB (β-hydroxybutyrate) to inhibit the NLRP3 inflammasome and decrease IL-1β, and decreases expression of NLRP3, Caspase-1, ASC, and IL-1β protein, which are inflammatory factors, to improve inflammation appearing upon the onset of alopecia areata, while decreasing NKG2D expression causing hair follicle destruction, and thus can be usefully used for preventing or treating alopecia areata.

## Description

### [TECHNICAL FIELD]

The present disclosure relates to a pharmaceutical composition for use in preventing or treating alopecia areata, comprising enavogliflozin as an active ingredient.

### [BACKGROUND]

Alopecia areata is a chronic recurrent alopecia disease, which is a relatively common disease occurring in about 2% of the world population, and may occur at all ages in men and women. Alopecia areata is known to have a genetic predisposition and an autoimmune response as the most important pathogenesis, and may be applied by various other environmental factors. However, the exact pathogenesis has not been revealed until now, and its treatment method is also unclear.

The most important cause of alopecia areata is an immunological factor, and for reasons such as a certain stimulus, a T cell is activated to misrecognize a hair follicle as a foreign substance and attack it, thereby inducing an immune response to cause alopecia areata. A recent non-clinical study suggested a new mechanism that alopecia areata occurs through an immune response due to NLRP3 inflammasome activation as a pathogenesis of alopecia areata, and an increase in expression of NLRP3, ASC, Caspase-1, and IL-1β constituting the NLRP3 inflammasome in a scalp hair follicle of a patient with alopecia areata has been reported (see Non-Patent Document 1, [JM Shin et al., Scientific Reports (2017) 7:44127]). Particularly, in an alopecia areata mouse model, a study confirmed that administration of an MCC950 substance known as an NLRP3 inhibitor inhibits NLRP3 inflammasome activity to alleviate an inflammatory response and also improve alopecia areata (see Non-Patent Document 2, [K Hashimoto et al., Exp Dermatol. 2022 Feb;31(2):133-142]).

Currently, various therapeutic agents such as a steroid agent and a janus kinase (JAK) inhibitor are used to treat alopecia areata. However, the necessity of a new therapeutic agent is emerging due to the time taken for an effect to appear, high cost, a side effect that may occur for a long-term intake, and a recurrence rate occurring when the drug is stopped.

Sodium-glucose co-transporter (hereinafter, SGLT) is a glucose transport protein, wherein SGLT-1 is expressed in the small intestine, liver, kidney, and heart, and SGLT-2 is mainly expressed in the kidney. An SGLT-2 inhibitor is a new class of anti-diabetic drug that reduces glucose reabsorption in the proximal nephron to increase glucose excretion through an insulin-independent mechanism. One of the drugs developed as an SGLT-2 inhibitor that may be usefully used as a therapeutic agent for type 2 diabetes due to such an effect is enavogliflozin, which has been disclosed in Korean Patent Application Publication No. 2014-0022086 (Patent Document 1).

In the course of research on another medical use of enavogliflozin, the present inventors noted that β-hydroxybutyrate (hereinafter, BHB) increases when fasting for 24 hours after taking enavogliflozin in healthy individual and type 2 diabetes clinical trials.

Recently, Non-Patent Document 3 has reported that an SGLT2 inhibitor attenuates NLRP3 inflammasome activation and IL-1β secretion affecting both type 2 diabetes and cardiovascular disease through an increase in serum BHB and a decrease in serum insulin (see Non-Patent Document 3, [SR Kim et al., Nature Communications (2020) 11:2127]).

The relationship between an increase in BHB and alopecia areata has not been reported yet. However, based on previously reported literatures, independently of blood glucose control of an SGLT-2 inhibitor, the present inventors focused research on the possibility that administration of an SGLT-2 inhibitor increases BHB, thereby inhibiting NLRP3 inflammasome activation to control inflammation, thereby preventing or treating alopecia areata.

### [Prior Art Document]

### [Patent Document]

Korean Patent Application Publication No. 2014-0022086

### [Non-Patent Document]

JM Shin et al., Scientific Reports (2017) 7:44127
K Hashimoto et al., Exp Dermatol. 2022 Feb;31(2):133-142
SR Kim et al., Nature Communications (2020) 11:2127

### [DESCRIPTION OF THE INVENTION]

### [TECHNICAL PROBLEM]

As a result of the above research, the present inventors completed the present disclosure by confirming that enavogliflozin has an excellent effect on the prevention or treatment of alopecia areata by improving inflammation through inhibition of NLRP3 inflammasome activation.

Accordingly, an object of the present disclosure is to provide a pharmaceutical composition for use in preventing or treating alopecia areata, comprising enavogliflozin as an active ingredient.

Another object of the present disclosure is to provide a method for preventing or treating alopecia areata, comprising administering a pharmaceutical composition for preventing or treating alopecia areata comprising enavogliflozin as an active ingredient to a subject in need thereof.

Still another object of the present disclosure is to provide a use of enavogliflozin for manufacturing a pharmaceutical composition for preventing or treating alopecia areata.

### [TECHNICAL SOLUTION]

The present disclosure relates to a pharmaceutical composition for use in preventing or treating alopecia areata comprising enavogliflozin as an active ingredient, and a prevention or treatment of alopecia areata using the same, and it was confirmed that the pharmaceutical composition according to the present disclosure is significantly excellent in preventing or treating alopecia areata.

Hereinafter, the present disclosure will be described in more detail.

One example of the present disclosure provides a pharmaceutical composition for use in preventing or treating alopecia areata, comprising enavogliflozin as an active ingredient.

In the following example, it was confirmed that administration of enavogliflozin tends to increase a plasma BHB concentration, and decreases IL-1β secreted when the NLRP3 inflammasome is activated. In addition, enavogliflozin decreased the expression of NLRP3, Caspase-1, ASC, and IL-1β protein, which are inflammatory factors, in a dose-dependent manner, thereby confirming the possibility of improving inflammation appearing upon the onset of alopecia areata. In addition, it was confirmed that enavogliflozin can improve alopecia areata through a decrease in NKG2D expression, which is expressed in a pathogenic cell in an alopecia areata disease and causes hair follicle destruction.

As used herein, the term "enavogliflozin" is an SGLT-2 (Sodium-Glucose Cotransporter 2) inhibitor, which is a drug that selectively inhibits SGLT-2 involved in reabsorption of glucose in the kidney to prevent glucose from being absorbed into the body and excrete it into urine.

In the present disclosure, "enavogliflozin" has a structure of Chemical Formula 1 below.

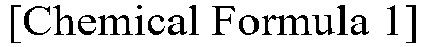

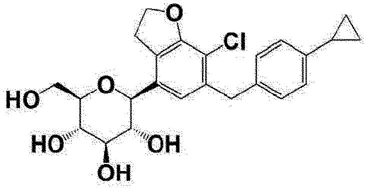

As used herein, the term "prevention" means all acts of suppressing or delaying the onset of alopecia areata by administration of the pharmaceutical composition according to the present disclosure.

As used herein, the term "treatment" means all acts of improving or beneficially changing alopecia areata by administration of the pharmaceutical composition according to the present disclosure.

Another example of the present disclosure is a method for preventing or treating alopecia areata, comprising administering a pharmaceutical composition for preventing or treating alopecia areata comprising enavogliflozin as an active ingredient to a subject in need thereof. In one example of the present disclosure, the pharmaceutical composition for preventing or treating alopecia areata comprising enavogliflozin as an active ingredient can be used in the form of single administration or co-administration.

Enavogliflozin may be used as a monotherapy for preventing or treating alopecia areata, but under various purposes, a therapeutic agent for alopecia areata is co-administered, and since it may exhibit a complementary or synergistic effect upon co-administration, the pharmaceutical composition comprising enavogliflozin can be co-administered with another therapeutic agent for alopecia areata.

In one example of the present disclosure, the pharmaceutical composition may be co-administered with one or more drugs selected from a corticosteroid, minoxidil, and a JAK inhibitor.

A corticosteroid may be frequently used for the treatment of mild to moderate alopecia areata. An administration route of the corticosteroid co-administered with enavogliflozin is not particularly limited, and may be an oral or parenteral corticosteroid.

Minoxidil, which is frequently prescribed for alleviating an alopecia symptom, may also be co-administered with enavogliflozin orally or parenterally.

A JAK inhibitor, which is a type of immunomodulator, is known to help suppress inflammation through cytokine reduction. A clinical report published in 2014 showed for the first time that Tofacitinib (product name Xeljanz^{®}), a JAK inhibitor, can successfully treat alopecia areata, and since then, many studies have shown that a JAK inhibitor may be effective in promoting hair regrowth in alopecia areata. Tofacitinib is still an old drug prescribed off-label for alopecia areata. From 2022, the FDA approved two JAK inhibitors, baricitinib and ritlecitinib, as a therapeutic agent for severe alopecia areata. Deuruxolitinib, another JAK inhibitor, is also undergoing clinical trials as a therapeutic agent for alopecia areata. Therefore, the JAK inhibitor that may be co-administered with enavogliflozin includes, but is not limited to, tofacitinib, baricitinib, ritlecitinib, deuruxolitinib, and the like.

In another example, the pharmaceutical composition of the present disclosure may be combined with immunotherapy for treating alopecia areata. Here, the immunotherapy for treating alopecia areata refers to topical immunotherapy, also called contact immunotherapy. Immunotherapy agents induce an allergic rash (allergic contact dermatitis) at an applied site, and although the exact reason is unknown, it is known to change a body immune response around a hair follicle and promote hair regrowth. A drug most commonly used for topical application to an alopecia areata site in immunotherapy, ie, an immunotherapy agent, is squaric acid dibutyl ester (SADBE) or diphencyprone (DPCP).

In one example of the present disclosure, the pharmaceutical composition may be for oral or parenteral use.

The pharmaceutical composition according to the present disclosure may further comprise a pharmaceutically acceptable carrier in addition to enavogliflozin of Chemical Formula 1, which is an active ingredient, or a pharmaceutically acceptable salt thereof, and may be formulated together with a carrier.

As used herein, the term "pharmaceutically acceptable carrier" refers to a carrier or diluent that does not irritate an organism and does not inhibit a biological activity and property of an administered compound. As a pharmaceutically acceptable carrier in a composition formulated into a liquid solution, which is sterile and biocompatible, saline, sterile water, Ringer's solution, buffered saline, albumin injection solution, dextrose solution, maltodextrin solution, glycerol, ethanol, and a mixture of one or more of these components may be used, and if necessary, other conventional additives such as an antioxidant, a buffer, and a bacteriostatic agent may be added. In addition, a diluent, a dispersant, a surfactant, a binder, and a lubricant may be additionally added to formulate into an injectable formulation such as an aqueous solution, a suspension, and an emulsion, a pill, a capsule, a granule, or a tablet.

In the present disclosure, the pharmaceutical composition may have a formulation for oral administration such as a tablet, a capsule, and the like. In one example of the present disclosure, the pharmaceutical composition may have a formulation of a tablet.

In the present disclosure, the pharmaceutical composition may have a formulation for parenteral administration. Although not limited thereto, for example, a formulation for parenteral administration comprising the composition of the present disclosure as an active ingredient may be formulated into an injection form such as a subcutaneous injection, an intravenous injection, or an intramuscular injection.

In order to formulate into an injectable formulation, the composition of the present disclosure may be mixed in water together with a stabilizer or a buffer to prepare a solution or a suspension, which may be formulated for unit dosage of an ampoule or a vial.

Alternatively, the composition of the present disclosure may be formulated into various forms of parenteral administration formulations, for example, an eye drop, a microneedle, a patch, a depot, and the like.

The composition of the present disclosure is administered in a pharmaceutically effective amount. In the present disclosure, "pharmaceutically effective amount" means an amount sufficient to treat a disease, and an effective dose level may be determined according to factors including a type of a patient's disease, severity, an activity of a drug, sensitivity to a drug, administration time, an administration route and excretion rate, a treatment period, a simultaneously used drug, and other factors well known in the medical field. The composition of the present disclosure may be administered as an individual therapeutic agent or administered in combination with another therapeutic agent, may be administered sequentially or simultaneously with a conventional therapeutic agent, and may be single or multiple administered. That is, a total effective amount of the composition of the present disclosure may be administered to a patient in a single dose, and may be administered by a fractionated treatment protocol in which multiple doses are administered for a long period of time. Considering all of the above factors, it is important to administer an amount that can obtain a maximum effect with a minimum amount without a side effect, which can be easily determined by those skilled in the art.

An appropriate once-daily administration dose of enavogliflozin identified during a clinical trial is 0.1 mg to 0.5 mg.

When the pharmaceutical composition is formulated into a unit dosage form, a content of the active ingredient in the pharmaceutical composition may be 0.1 mg to 0.5 mg.

The pharmaceutical composition according to the present disclosure may be administered 1 to 3 times a day, for example, once a day, but is not limited thereto.

In the present disclosure, a dose of enavogliflozin that can be used for preventing or treating alopecia areata is not particularly limited, and may be appropriately adjusted by severity of a disease of an administration subject, body weight, age, sex, and the presence or absence of other complications.

Since the method for preventing or treating alopecia areata uses a pharmaceutical composition for preventing or treating alopecia areata, overlapping contents between the two are omitted to avoid excessive description of the specification.

Enavogliflozin used as an active ingredient in the present disclosure can be synthesized through known prior literatures. In the present disclosure, enavogliflozin may be crystalline or amorphous. For example, enavogliflozin may be enavogliflozin crystalline form A, crystalline form B, crystalline form C, crystalline form D, crystalline form E, or enavogliflozin amorphous form reported to have the following X-ray diffraction spectrum through Korean Patent Application Publication No. 2017-0142904 or Korean Patent Application No. 2022-0123673.
Crystalline form A: a crystalline form having an X-ray diffraction (XRD) spectrum comprising peaks at 2-theta values selected from 6.2°±0.2°, 7.2°±0.2°, 8.8°±0.2°, 17.6°±0.2°, 19.0°±0.2°, 22.5°±0.2° and 25.1°±0.2°
Crystalline form B: a crystalline form having an X-ray diffraction (XRD) spectrum comprising peaks at 2-theta values selected from 7.0°±0.2°, 14.9°±0.2°, 17.7°±0.2°, 18.8°±0.2°, 20.6°±0.2°, 21.8°±0.2° and 23.5°±0.2°
Crystalline form C: a crystalline form having an X-ray diffraction (XRD) spectrum comprising peaks at 2-theta values selected from 5.6°±0.2°, 7.3°±0.2°, 15.7°±0.2°, 17.2°±0.2°, 18.9°±0.2°, 21.2°±0.2° and 21.9°±0.2°
Crystalline form D: a crystalline form having an X-ray diffraction (XRD) spectrum comprising peaks at 2-theta values selected from 5.5°±0.2°, 7.2°±0.2°, 15.3°±0.2°, 17.2°±0.2°, 17.6°±0.2°, 18.9°±0.2° and 21.1°±0.2°
Crystalline form E: a crystalline form having an X-ray diffraction (XRD) spectrum comprising peaks at 2-theta values selected from 4.93°±0.2°, 6.12°±0.2°, 7.43°± 0.2°, 8.89°± 0.2°, 9.74°± 0.2°, 14.79°± 0.2°, 15.79°± 0.2°, 16.11°± 0.2°, 19.79°± 0.2° and 22.83°±0.2°

Each of crystalline forms A, B, C, D, and E can be designated by an X-ray diffraction spectrum having 4 or more, for example, 4, 5, 6, 7, 8 or more peaks at the 2-theta values presented above.

Although not limited thereto, the pharmaceutical composition comprising enavogliflozin according to the present disclosure may have a configuration of a pharmaceutical composition of PCT/KR2022/014640.

For example, the pharmaceutical composition of the present disclosure may be a pharmaceutical composition comprising a compound of Chemical Formula 1 or a pharmaceutically acceptable salt thereof, an excipient, a disintegrant, and a binder as an active ingredient, wherein an average particle size of the compound of Chemical Formula 1 is 15 um or less.

In an example of the present disclosure, an average particle size of enavogliflozin may be 15 um or less, preferably 10 um or less.

When the average particle size of enavogliflozin exceeds 15 um, a 5-minute dissolution rate is very low as less than 40% of a total content of enavogliflozin, and a 30-minute dissolution rate is also less than 80%, indicating that a final dissolution rate is inappropriate.

When micronization of a drug particle size is required, it may be pulverized using a conventional mill capable of micronizing particles, such as a jet mill (Z-mill), a hammer mill, a ball mill, and a fluid energy mill. In addition, a particle size of a drug may be subdivided by using a size classification method such as a sieving method performed using a sieve or an air current classification. A method for controlling a desired particle size is well known in the art. See, for example, the following literature: [Pharmaceutical dosage forms: volume 2, 2nd edition, Ed.: H.A.Lieberman, L.Lachman, J.B.Schwartz (Chapter 3: SIZE REDUCTION)].

In the present specification, a particle size of a drug is expressed based on a particle size distribution such as D(X) = Y (where X and Y are positive numbers). D(X) = Y means that when a particle size distribution of a certain drug obtained by measuring a particle diameter of the drug in a formulation is represented by a cumulative curve, a particle diameter at a point where X% (% is calculated based on number, volume, or weight) is accumulated in an order of smaller particle size is Y. For example, D(10) represents a diameter of a particle at a point where a particle size of a drug is accumulated to 10% in an order of smaller size, D(50) represents a diameter of a particle at a point where a particle size of a drug is accumulated to 50% in an order of smaller size, and D(90) represents a diameter of a particle at a point where a particle size of a drug is accumulated to 90% in an order of smaller size.

Whether the particle size distribution D(X) represents a percentage of total cumulative particles based on number, volume, or weight depends on a method used to measure the particle size distribution. A method for measuring a particle size distribution and a type of % associated therewith are known in the art. For example, when a particle size distribution is measured by a well-known laser diffraction method, an X value in D(X) represents a percentage calculated by a volume average. Those skilled in the art well know that a particle size distribution measurement result obtained by a specific method may be correlated with that obtained from another technique based on experience through routine experimentation. For example, a laser diffraction method provides a volume average particle size in response to a volume of a particle, which corresponds to a weight average particle size when a density is constant.

In the present disclosure, measurement of a particle size distribution of a drug particle can be performed using a commercially available apparatus based on a laser diffraction/scattering method based on Mie theory. For example, measurement is performed using a commercially available apparatus such as a Mastersizer laser diffraction apparatus manufactured by Malvern Instruments. This apparatus irradiates a particle with a helium-neon laser beam and a blue light-emitting diode to cause scattering, whereby a light scattering pattern appears on a detector, and a particle diameter distribution is obtained by analyzing the light scattering pattern according to Mie theory. The measurement method may be either a dry method or a wet method. In one embodiment of the present disclosure, an average particle size of enavogliflozin may be a volume average particle size by a laser diffraction method.

In an example of the present disclosure, the compound of Chemical Formula 1 may be included in an amount of less than 1 part by weight based on 100 parts by weight of a total pharmaceutical composition.

An appropriate once-daily administration dose of enavogliflozin identified during a clinical trial is 0.1 mg to 0.5 mg, and when the pharmaceutical composition is formulated into a unit dosage form, a content of the active ingredient in the pharmaceutical composition may be 0.1 mg to 0.5 mg.

The pharmaceutical composition according to the present disclosure includes pharmaceutically acceptable additives in addition to the compound of Chemical Formula 1, which is an active ingredient.

The pharmaceutical composition of the present disclosure includes an excipient, a disintegrant, a binder, and the like as an additive.

Examples of the excipient include lactose (including a hydrate), dextrin, mannitol, sorbitol, starch, microcrystalline cellulose [for example, CelphereTM], silicified microcrystalline cellulose [for example, ProsolvTM], calcium phosphate hydrate, anhydrous calcium phosphate, calcium carbonate, sugars, or a mixture thereof. In an example of the present disclosure, a preferred excipient is microcrystalline cellulose.

Examples of the disintegrant include crospovidone, croscarmellose sodium, sodium starch glycolate, and low-substituted hydroxypropyl cellulose. In an example of the present disclosure, a preferred excipient is croscarmellose sodium.

Examples of the binder include polyvinylpyrrolidone, povidone, gelatin, starch, sucrose, methylcellulose, ethylcellulose, hydroxyethylcellulose, hydroxypropylcellulose, hydroxypropylalkylcellulose (for example, hydroxypropylmethylcellulose), and a mixture thereof. In an example of the present disclosure, a preferred binder is hydroxypropylcellulose.

Examples of other additives include a lubricant, a coloring agent, and the like.

The lubricant includes stearic acid, stearate (for example, magnesium stearate), light anhydrous silicic acid, talc, corn starch, carnauba wax, magnesium silicate, synthetic aluminum silicate, hydrogenated oil, white wax, titanium oxide, microcrystalline cellulose, macrogol 4000 and 6000, isopropyl myristate, calcium hydrogen phosphate, and a mixture thereof.

In an example of the present disclosure, the excipient may be included in an amount of 80 to 95 parts by weight based on 100 parts by weight of a total pharmaceutical composition.

In an example of the present disclosure, the disintegrant may be included in an amount of 2 to 8 parts by weight based on 100 parts by weight of a total pharmaceutical composition. When the disintegrant is less than 2 parts by weight based on 100 parts by weight of the total pharmaceutical composition, an initial disintegrating force is low, so that a dissolution rate may be delayed, which may affect an in vivo Cmax. In addition, when it exceeds 8 parts by weight based on 100 parts by weight of the total pharmaceutical composition, an amount of the disintegrant in a post-mixing part increases, so that overall flowability of a granule may be deteriorated.

In an example of the present disclosure, the binder may be included in an amount of 3 to 10 parts by weight based on 100 parts by weight of a total pharmaceutical composition. When the binder is less than 3 parts by weight based on 100 parts by weight of the total pharmaceutical composition, it may be difficult to form and maintain a suitable dry granule, which may affect maintenance of homogeneous dispersibility of a main ingredient and granule flowability due to generation of fine powder. In addition, when it exceeds 10 parts by weight based on 100 parts by weight of the total pharmaceutical composition, a granule having a strong binding force is formed, which affects solubility of initially disintegrated granule particles during dissolution, which may also affect an in vivo Cmax.

The pharmaceutical composition according to the present disclosure may be an immediate-release formulation.

In one example of the present disclosure, the pharmaceutical composition may have a dissolution rate after 5 minutes of 50% or more, preferably 60% or more of a total content of the active ingredient.

In one example of the present disclosure, the pharmaceutical composition may have a dissolution rate after 15 minutes of 80% or more, preferably 80% or more of a total content of the active ingredient.

In one example of the present disclosure, the pharmaceutical composition may have a dissolution rate after 30 minutes of 85% or more, preferably 90% or more of a total content of the active ingredient.

Since a dissolution rate of an active ingredient in a pharmaceutical composition affects a maximum blood concentration (Cmax) and an area under a blood concentration-time curve (AUC) upon drug administration, conversely speaking, it is important to adjust a dissolution rate of a pharmaceutical composition to implement an appropriate Cmax and AUC. Since enavogliflozin has a Tmax of 1 to 2 hours, a drug absorption rate in the stomach is judged to be important. The dissolution rate is measured under a condition of a dissolution medium 1.2 by a Korean Pharmacopoeia Dissolution Test Method 2 (Paddle Method). Specific conditions may refer to the following experimental examples.

The present disclosure also provides a pharmaceutical composition comprising a granulated product in which a pre-mix granule comprising a compound of Chemical Formula 1 or a pharmaceutically acceptable salt thereof and a post-mixing part are mixed.

In the course of research on formulation of the compound of Chemical Formula 1, the present inventors confirmed that manufacturing a granulated product and formulating it into a tablet or the like is advantageous in terms of content uniformity and dosage unit uniformity of a drug.

In the pharmaceutical composition, the granulated product is manufactured by mixing a pre-mix granule and a post-mixing part.

The pre-mix granule may include a compound of Chemical Formula 1 or a pharmaceutically acceptable salt thereof, an excipient, a binder, and a lubricant.

In addition, the post-mixing part may include an excipient, a disintegrant, and a lubricant.

Descriptions of an excipient, a binder, a disintegrant, a lubricant, and the like are the same as those described above, and thus overlapping descriptions are omitted to avoid repetition.

In an example of the present disclosure, the pre-mix granule and the post-mixing part may each include an excipient. More specifically, the pre-mix granule and the post-mixing part may each include microcrystalline cellulose as an excipient.

It was identified that microcrystalline cellulose included in the pre-mix granule and the post-mixing part affects content uniformity of a drug depending on a particle size and bulk density thereof.

In an example of the present disclosure, a particle size of microcrystalline cellulose in the pre-mix granule may be 130 um or less, preferably 60 um to 130 um. A bulk density of microcrystalline cellulose in the pre-mix granule may be 0.26 to 0.33. When a particle size and bulk density of microcrystalline cellulose in a pre-mix granule are under such conditions, a formulation having a low standard deviation (SD) of content uniformity can be secured. When a particle size of microcrystalline cellulose in a pre-mix granule is 130 um or less, content uniformity of a pre-mix part granule, content uniformity of a final granule, and a formulation all showed a good level, and a Carr's index value representing physical properties of a final granule was also good, indicating that flowability of a formulation was also excellent. On the other hand, when a particle size of microcrystalline cellulose in a pre-mix granule exceeds 130 um, a deviation in both content uniformity of a pre-mix part granule and content uniformity of a final granule increased, which was inappropriate, and dosage unit uniformity was also found to be poor.

Meanwhile, a particle size of microcrystalline cellulose in the post-mixing part may be 130 um or more, preferably 130 um to 250 um. A bulk density of an excipient in the post-mixing part may be 0.28 to 0.37. When a particle size of microcrystalline cellulose in the post-mixing part is less than 130 um, a Carr's index value representing physical properties of a final granule was not appropriate, and it could be confirmed that granule flowability became weak.

Comparing microcrystalline cellulose in a pre-mix granule and microcrystalline cellulose in a post-mixing part relatively, unlike that a small particle size of microcrystalline cellulose included in a pre-mix granule is preferable, a particle size of microcrystalline cellulose of a post-mixing part was found to be preferably relatively larger than a particle size of microcrystalline cellulose included in a pre-mix granule.

It was confirmed that not only a particle size of microcrystalline cellulose in a pre-mix granule and microcrystalline cellulose in a post-mixing part, but also a weight ratio of an excipient in a pre-mix granule and an excipient in a post-mixing part affects content uniformity of a drug.

In an example of the present disclosure, a weight ratio of an excipient in the pre-mix granule and an excipient in the post-mixing part may be 4:1 to 1:1. It was identified that as a ratio of microcrystalline cellulose in a post-mixing part increases, flowability of a granule becomes excellent while a content deviation increases, so that it is preferable to match a weight ratio in the appropriate range.

Meanwhile, in the pharmaceutical composition according to the present disclosure, the binder may be one or more selected from the group consisting of hydroxypropylcellulose, povidone, copovidone, and hypromellose.

In one example of the present disclosure, the binder is hydroxypropylcellulose, and a weight average molecular weight may be less than 200,000. When hydroxypropylcellulose having a weight average molecular weight of 200,000 or more is used, both a 5-minute dissolution rate and a 30-minute dissolution rate are low, which is disadvantageous in terms of bioavailability.

On the other hand, in relation to a Carr's index used as a measure of flowability in formulation, a Carr's index of the granulated product is preferably 21 to 25.

Although not limited thereto, in the pharmaceutical composition of the present disclosure, the granulated product may be a dry granulated product. In another example, the granulated product may be a wet granulated product.

In the present disclosure, the pharmaceutical composition may have a formulation for oral administration such as a tablet, a capsule, and the like. In one example of the present disclosure, the pharmaceutical composition may have a formulation of a tablet.

In a preferred example, the pharmaceutical composition may include the compound of Chemical Formula 1 in a dose of 0.3 mg.

The pharmaceutical composition according to the present disclosure may be orally administered once a day, but is not limited thereto.

### [EFFECT OF INVENTION]

Enavogliflozin of the present disclosure increases BHB (β-hydroxybutyrate) to inhibit the NLRP3 inflammasome and decrease IL-1β, and decreases expression of NLRP3, Caspase-1, ASC, and IL-1β protein, which are inflammatory factors, to improve inflammation appearing upon the onset of alopecia areata, while decreasing NKG2D expression causing hair follicle destruction, and thus can be usefully used for preventing or treating alopecia areata.

### [DESCRIPTION OF DRAWINGS]

FIG. 1 is a graph showing a ratio of a number of mice in which alopecia areata did not occur according to an example of the present disclosure.
FIG. 2 is a graph showing a ratio of a size in which alopecia areata occurred in a mouse in which alopecia areata occurred according to an example of the present disclosure.
FIG. 3 is a diagram showing a representative comparative photograph for each substance-treated group of a mouse in which alopecia areata occurred according to an example of the present disclosure.
FIG. 4 is a graph showing BHB production confirmed in plasma of a mouse in which alopecia areata occurred according to an example of the present disclosure.
FIG. 5 is a diagram showing a change in protein expression of an inflammatory factor through staining in a skin tissue of a mouse in which alopecia areata occurred according to an example of the present disclosure.
FIG. 6 is a diagram showing a change in protein expression of an inflammatory factor in a lymph node cell of a mouse in which alopecia areata occurred according to an example of the present disclosure.
FIG. 7 is a diagram showing a change in expression of NKG2D confirmed upon the onset of alopecia areata in a pathogenic cell isolated from a lymph node cell of a mouse in which alopecia areata occurred according to an example of the present disclosure.

### [MODE FOR INVENTION]

Advantages and features of the present disclosure, and a method of achieving them will become clear with reference to examples described in detail below. However, the present disclosure is not limited to examples disclosed below, but will be implemented in various different forms, and only the present examples are provided to make a disclosure of the present disclosure complete, and to completely inform those skilled in the art to which the present disclosure belongs of a scope of the disclosure, and the present disclosure is only defined by a scope of a claim.

### Example 1. Evaluation of alopecia areata efficacy of enavogliflozin in alopecia areata mouse model.

An 8-week-old C3H/Hej female mouse was obtained, sufficiently supplied with solid feed (without antibiotics) and water until an experiment day, adapted in an environment of a temperature of 23±2°C, humidity of 55±10%, and a 12-hours light-dark cycle, and then used in an experiment.

An alopecia areata model was manufactured using a lymph node cell injection method. A lymph node cell was isolated from a mouse (donor) in which alopecia areata occurred, proliferated using Dynabead Mouse T-activator CD3/CD28, Human Recombinant IL-2, Mouse Recombinant IL-7, and Mouse Recombinant IL-15, and then injected into a C3H/Hej mouse (recipient) of 10 weeks of age or older by 1.5x10⁷ cells/mouse intra-dermal injection to manufacture an alopecia areata model.

In order to evaluate alopecia areata efficacy of enavogliflozin in an alopecia areata mouse model manufactured by a lymph node cell injection method, a test group was set and evaluated as shown in Table 1 below.

**[Table 1]**

| Test Group | Method for Inducing Alopecia Areata | Test Substance | Dose (mg/kg) | Route of Administration | Number of Administrations | Number of Mice |
|---|---|---|---|---|---|---|
| G1 | Lymph node cell injection method | Vehicle (NC) | N/A | PO | QD | 6 |
| G2 | | Enavogliflozin (ENA) | 0.1 | | | 6 |
| G3 | | | 1 | | | 6 |

As a test substance, enavogliflozin 0.1 mg/kg and 1 mg/kg were used. A vehicle group was prepared in 0.5% Carboxymethyl cellulose sodium salt with 1% Tween80, stored at 4°C, and used.

At 4 weeks after injecting a lymph node cell into a mouse, upon visual confirmation of alopecia areata, individuals were selected and divided into groups, and administration of a test substance was started. Individual selection was performed by selecting a mouse in which alopecia areata occurred by 50% or more, a mouse in which alopecia areata occurred by 50% or less, and a mouse in which alopecia areata did not occur at a time when alopecia areata was induced in about 50% of total mice after lymph node cell administration, so that a total of 6 mice were included in each group.

An administration period of a test substance is 12 weeks, and a vehicle group and an enavogliflozin group were orally administered (P.O. injection) once every day (QD).

### (1) Measurement of disease-free ratio

An alopecia areata incidence rate expressed a ratio of a number of mice in which alopecia areata did not occur to a total number of individuals as %.

Prism 10.0 software (GraphPad Software Inc., San Diego, CA, USA) was used for statistical analysis of data. Experimental results were expressed as mean±standard error of the mean (SEM). As a statistical method, a one-way analysis of variance (One-way ANOVA) was performed to verify a difference between each group, and a post hoc test for each type used a verification method through a Tukey function (Tukey's test) or Dunn's multiple comparison verification method (Dunn's multiple comparison test). A significance level was set to p<0.05.

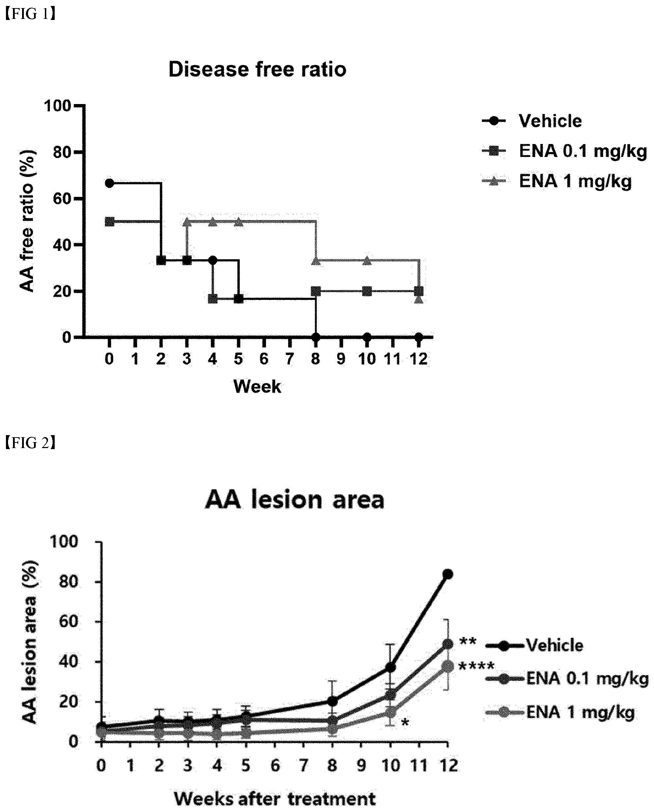

FIG. 1 and Table 2 show a ratio of a number of mice in which alopecia areata did not occur according to an example of the present disclosure.

**[Table 2]**

| Disease-free ratio | | | |
|---|---|---|---|
| Week | Vehicle | ENA 0.1 mg/kg | ENA 1 mg/kg |
| 0 | 4/6 (66.7%) | 3/6 (50%) | 3/6 (50%) |
| 2 | 2/6 (33.3%) | 2/6 (33.3%) | 2/6 (33.3%) |
| 3 | 2/6 (33.3%) | 2/6 (33.3%) | 3/6 (50%) |
| 4 | 2/6 (33.3%) | 1/6 (16.7%) | 3/6 (50%) |
| 5 | 1/6 (16.7%) | 1/6 (16.7%) | 3/6 (50%) |
| 8 | 0/5 (0%) | 1/5 (20%) | 2/6 (33.3%) |
| 10 | 0/5 (0%) | 1/5 (20%) | 2/6 (33.3%) |
| 12 | 0/5 (0%) | 1/5 (20%) | 1/6 (16.7%) |

As can be confirmed in FIG. 1 and Table 2, in a vehicle group, alopecia areata appeared 100% in all individuals at 12 weeks (8 weeks of substance administration) after lymph node cell injection into a mouse. In an enavogliflozin-administered group, alopecia areata appeared 80 to 83.3% until 16 weeks (12 weeks of substance administration) after lymph node cell injection. These results mean that enavogliflozin protects or improves occurrence of alopecia areata.

### (2) Measurement of AA lesion size

A size of alopecia areata was photographed once every 2 weeks using a digital camera (DSLR), and a ratio of an area where a symptom occurred to a total area of an individual was expressed as % by image J (National Institutes of Health). Occurrence of alopecia areata measures a whole back and belly of a mouse.

Prism 10.0 software (GraphPad Software Inc., San Diego, CA, USA) was used for statistical analysis of data. Experimental results were expressed as mean±standard error of the mean (SEM). As a statistical method, a one-way analysis of variance (One-way ANOVA) was performed to verify a difference between each group, and a post hoc test for each type used a verification method through a Tukey function or Dunn's multiple comparison verification method. A significance level was set to p<0.05.

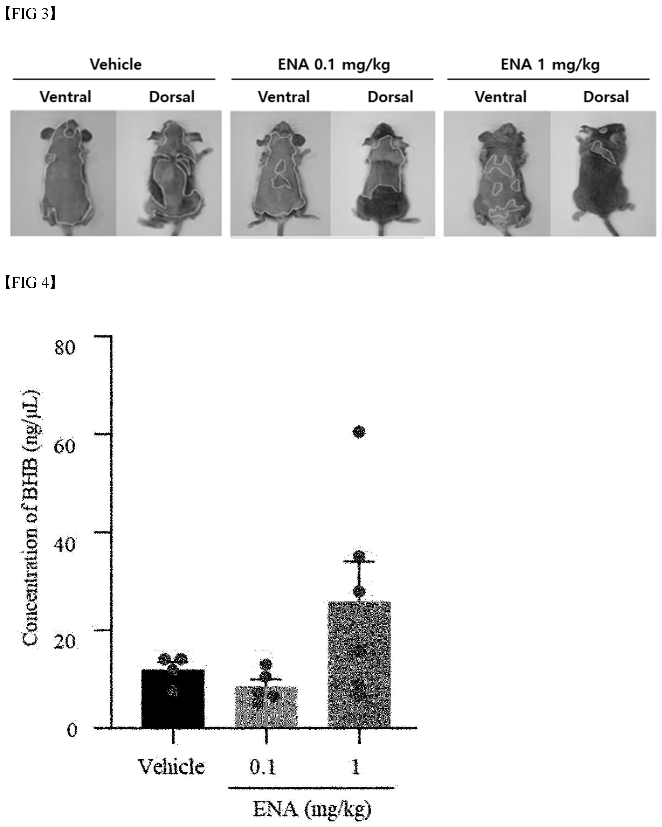

As can be seen from FIG. 2 and FIG. 3, at 12 weeks of final administration, it was confirmed that a size in which alopecia areata occurred was small in an order of an enavogliflozin 1 mg/kg administration group and an enavogliflozin 0.1 mg/kg administration group compared to a vehicle group. In the enavogliflozin 1 mg/kg administration group, a significant difference was shown compared to the vehicle group. These results indicate that enavogliflozin protects or improves alopecia areata from being developed.

### (3) Measurement of Plasma BHB concentration

After a test was completed, fasting was induced by removing feed for 24 hours before collecting blood. Collected blood was placed in a tube treated with an anticoagulant and centrifuged at 13,000 rpm for 10 minutes to obtain a supernatant. Thereafter, a protein in blood was concentrated with a 10 kDa molecular weight cut-off (MWCO) spin filter, and a BHB concentration was confirmed using a BHB assay kit (MAK041, Sigma, MO, USA).

Prism 10.0 software (GraphPad Software Inc., San Diego, CA, USA) was used for statistical analysis of data. Experimental results were expressed as mean±standard error of the mean (SEM). As a statistical method, a one-way analysis of variance (One-way ANOVA) was performed to verify a difference between each group, and a post hoc test for each type used a verification method through a Tukey function or Dunn's multiple comparison verification method. A significance level was set to p<0.05.

As can be seen from FIG. 4, although there was no statistical significance compared to a vehicle group, a trend of increasing a plasma BHB concentration by administration of enavogliflozin 1 mg/kg was confirmed.

### (4) Histological analysis

At 12 weeks, which is a time point of completion of test substance administration, skin was excised and then fixed in a 10% formalin solution. After manufacturing a paraffin block, a tissue was cut to manufacture a slide, and then stained using an ImmPRESS Anti-Rabbit Ig Kit (Catalog No. MP-7401) or an ImmPRESS Anti-Rat Ig Kit (Catalog No. MP-7404). As a primary antibody, NLRP3 (768319, Invitrogen, CA, USA), caspase1 (Ab138483, Abcam, Cambridge, UK), ASC (NBP1-78977, Novusbio, CO, USA), and IL-1β (AF5103, Affinity biosciences, OH, USA) were used. For reading of a tissue, a tissue slide was photographed at 100 magnification using an optical microscope (PANORAMIC MIDI II), and then an expression level of each slide was read using Case Viewer software.

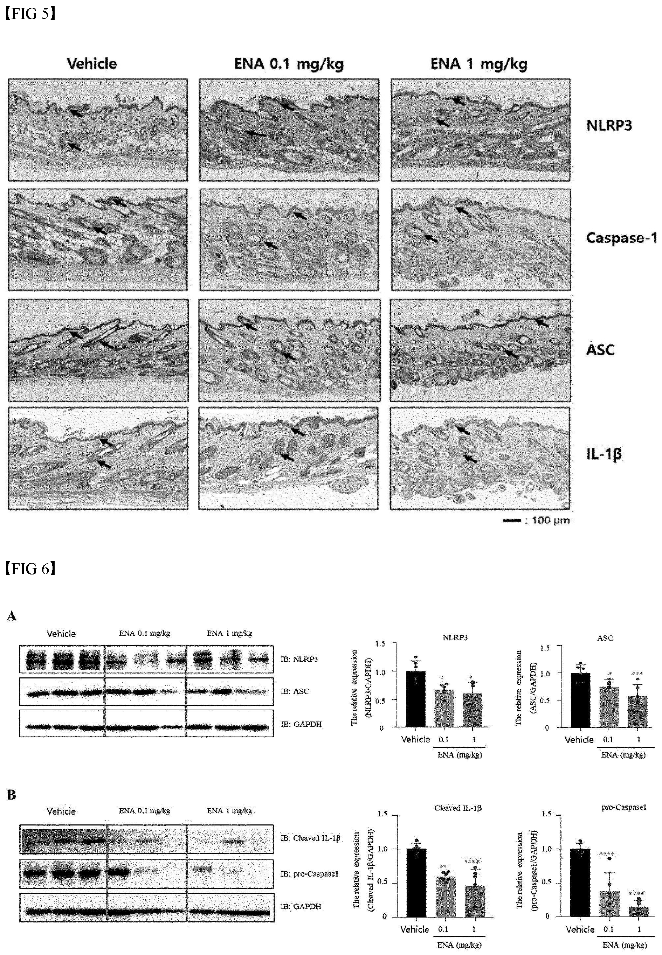

As can be seen from FIG. 5, in a vehicle group, it can be confirmed that an epidermis, dermis, and hair follicle vicinity of skin are stained brown, which means that NLRP3, Caspase-1, ASC, which are constituent factors of the NLRP3 inflammasome, an inflammation target, and IL-1β secreted when the NLRP3 inflammasome is activated are highly expressed in skin of an alopecia areata mouse model. It was confirmed that expression of inflammatory factors was decreased in an enavogliflozin 1 mg/kg administration group compared to the vehicle group, indicating that inflammation related to the NLRP3 inflammasome can be improved in skin of an alopecia areata mouse model.

### (5) Analysis of change in inflammatory factor protein expression

At 12 weeks, which is a time point of completion of test substance administration, skin was excised and lysed, and then a protein concentration was measured using a BCA protein assay kit (Bio-Rad, Hercules, CA, USA). A protein (20 µg) was separated by SDS-PAGE (polyacrylamide gel electrophoresis) and adsorbed onto a PVDF (polyvinylidene fluoride) membrane (Millipore Corp, Bedford, MA). A membrane was reacted in PBS containing 0.1% Tween-20 and 5% skim milk (non-fat milk), and reacted using a primary antibody diluted with 0.2% BSA (in PBS). After washing 3 times, a membrane was reacted with an HRP-conjugated secondary antibody (anti-rabbit IgG antibody (PI-1000) or anti-mouse IgG antibody (PI-2000), Vector Labs Inc., Burlingame, CA, USA), and a band was visualized with an ECL advance kit (ATTO, Tokyo, Japan).

Prism 10.0 software (GraphPad Software Inc., San Diego, CA, USA) was used for statistical analysis of data. Experimental results were expressed as mean±standard error of the mean (SEM). As a statistical method, a one-way analysis of variance (One-way ANOVA) was performed to verify a difference between each group, and a post hoc test for each type used a verification method through a Tukey function or Dunn's multiple comparison verification method. A significance level was set to p<0.05.

As can be seen from FIG. 6, it was confirmed that NLRP3, Caspase-1, ASC, and IL-1β protein, which are inflammatory factors, were significantly decreased in a dose-dependent manner in enavogliflozin 0.1 and 1 mg/kg treated groups compared to a vehicle group. Based on FIG. 5 and FIG. 6, it can be confirmed that as expression of an inflammatory factor decreases upon enavogliflozin treatment in a skin tissue in an alopecia areata mouse model, inflammation appearing upon the onset of alopecia areata can be improved.

### (6) Flow cytometry analysis of lymph node

Skin-Draining Lymph Node cells of a mouse obtained at a time point of completion of a test were taken out of a nitrogen tank and used for flow cytometry analysis. Cells were analyzed by staining antibodies of L/D, CD3, CD4, CD8, CD44, CD49d, CD62L, and NKG2D without additional stimulation. A cell surface protein was quantitatively analyzed for stained cells using a FACSymphony A1 (Becton, Dickinson and Company, BD) apparatus. Thereafter, MFI and a positive cell population were confirmed using FlowJo (Becton, Dickinson and Company, BD, v. 10.0).

Experimental results were expressed as mean±standard error of the mean (SEM). GraphPad Prism version 7 software was used to confirm statistical significance. Significance verification of flow cytometry data used a Mann-Whitney test, and it was considered statistically significant when a p value was 0.05 or less.

It is known that upon the onset of alopecia areata, a virtual memory T cell (TVM cell) becomes a pathogenic cell by an inflammatory cytokine, and NKG2D expression is upregulated by such a cytokine to destroy a hair follicle, thereby contributing to aggravation of a symptom of alopecia areata (see Non-Patent Document 5, [Seok J et al., Nature Immunology 24, 1308-1317 (2023)]).

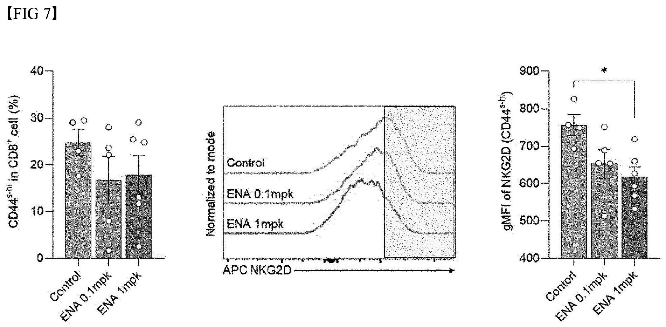

As can be seen from FIG. 7, a ratio of a pathogenic cell (CD44s-hi) in a lymph node cell did not show a significant ratio decrease in an enavogliflozin-administered group compared to a vehicle group, but an MFI value of NKG2D expressed upon the onset of alopecia areata in a pathogenic cell showed a significant decrease at enavogliflozin 1 mg/kg. This indicates that enavogliflozin can improve alopecia areata through a decrease in NKG2D expression, which is expressed in a pathogenic cell in an alopecia areata disease and causes hair follicle destruction.

## Claims

1. A pharmaceutical composition for use in preventing or treating alopecia areata, comprising enavogliflozin as an active ingredient.

2. The pharmaceutical composition according to claim 1, wherein the pharmaceutical composition is for oral or parenteral administration.

3. The pharmaceutical composition according to claim 1, wherein a single dose of the enavogliflozin is 0.1 mg to 0.5 mg.

4. The pharmaceutical composition according to claim 1, wherein the pharmaceutical composition is administered once a day.

5. The pharmaceutical composition according to claim 1, wherein the pharmaceutical composition is co-administered with one or more drugs selected from a corticosteroid, minoxidil, and a JAK inhibitor.

6. The pharmaceutical composition according to claim 1, wherein the pharmaceutical composition is used in combination with immunotherapy for treating alopecia areata.
